# EUROPEAN PATENT SPECIFICATION

(11) **EP 3 978 053 B1**
(45) Date of publication and mention of the grant of the patent: **08.10.2025**
(21) Application number: 19931290.1
(22) Date of filing: 27.12.2019
(51) Int. Cl.: A61M 15/00, A61F 7/03

(54) **STEAM GENERATION BODY, HEATING IMPLEMENT, AND PRODUCTION METHOD FOR STEAM GENERATION BODY**
DAMPFERZEUGUNGSKÖRPER, HEIZGERÄT UND HERSTELLUNGSVERFAHREN FÜR EINEN DAMPFERZEUGERKÖRPER
CORPS DE GÉNÉRATION DE VAPEUR, ACCESSOIRE DE CHAUFFAGE, ET PROCÉDÉ DE PRODUCTION DE CORPS DE GÉNÉRATION DE VAPEUR

(30) Priority: 24.05.2019 JP 2019097618; 09.10.2019 JP 2019185896
(43) Date of publication of application: 06.04.2022
(73) Proprietor: Kao Corporation, Chuo-Ku Tokyo 103-8210 (JP)
(72) Inventor: OKAMOTO Takuya, Tokyo 131-8501 (JP); TSUCHIYA Shigemi, Tokyo 131-8501 (JP)
(74) Representative: Hoffmann Eitle
(86) International application number: PCT/JP2019/051350
(87) International publication number: WO 2020/240903

(56) References cited:
- WO-A1-2017/217401
- JP-A- 2001 000 230
- JP-A- 2017 225 813
- US-A1- 2018 209 637

## Description

### Technical Field

The present invention relates to a steam generator that generates steam by heat generated by an oxidation reaction of an oxidizable metal, a heating tool including the steam generator, and a method for producing the steam as required by the present claims.

### Background Art

Conventionally, various heating tools using heat generated by an oxidation reaction of an oxidizable metal have been developed, and for example, there are heating tools that generate steam by the generated heat to wet a throat and a nose. Examples of the steam generator used for such a heating tool include a steam generator in which a heat generating layer formed of a heat generating composition containing an oxidizable metal, water, and a water retention agent and a polymer sheet (water retention sheet) in which a water absorbing polymer is interposed between water absorbing sheets are laminated (Patent Literature 1) and a steam generator including a heat generating layer containing an oxidizable metal, a carbon component, a water absorbing polymer, and water (patent literature 2). US2019732216 discloses a steam generator.

### Citation List

### Patent Literature

Patent Literature 1: JP 2013-146554 A
Patent Literature 2: JP 2017-23712 A

### Summary of Invention

### Technical Problem

In recent years, an awareness of health or prevention has increased, and there is a demand for a heating tool that can generate a large amount of steam in a shorter time. However, it is found that a conventional heating tool cannot satisfy the demand of consumers in recent years.

The present invention relates to a steam generator that can generate a large amount of steam in a short time.

### Solution to Problem

The present invention relates to a steam generator comprising a heat generating layer comprising an oxidizable metal, a carbon component, and water, a first water absorbent layer laminated on the heat generating layer and comprising a first water absorbent, a water absorbing sheet laminated on the first water absorbent layer, and a second water absorbent layer laminated on the water absorbing sheet and comprising a second water absorbent.

### Advantageous Effects of Invention

The steam generator according to the present invention can generate a large amount of steam in a short time.

### Brief Description of Drawings

Fig. 1 is a schematic view illustrating a configuration of a cross section of a steam generator of the present embodiment.
Fig. 2 is a schematic view illustrating a configuration of a cross section of a steam generating sheet used in an embodiment.
Fig. 3 is a view for describing a method for producing a steam generating sheet used in an embodiment.
Fig. 4 is a perspective view for describing a usage example of a heating tool.
Fig. 5 is a development view of a main body portion for describing the heating tool of the present embodiment.
Fig. 6 is a schematic view illustrating an air permeation resistance measuring device.
Fig. 7 is a schematic view illustrating a configuration of a cross section of a steam generating sheet used in a comparative example.
Fig. 8 is a schematic view illustrating a configuration of a cross section of a steam generating sheet used in a comparative example.
Fig. 9 is a schematic view illustrating a configuration of a cross section of a steam generating sheet used in a comparative example.
Fig. 10 is a schematic view illustrating a device measuring the amount of steam generated.

### Description of Embodiments

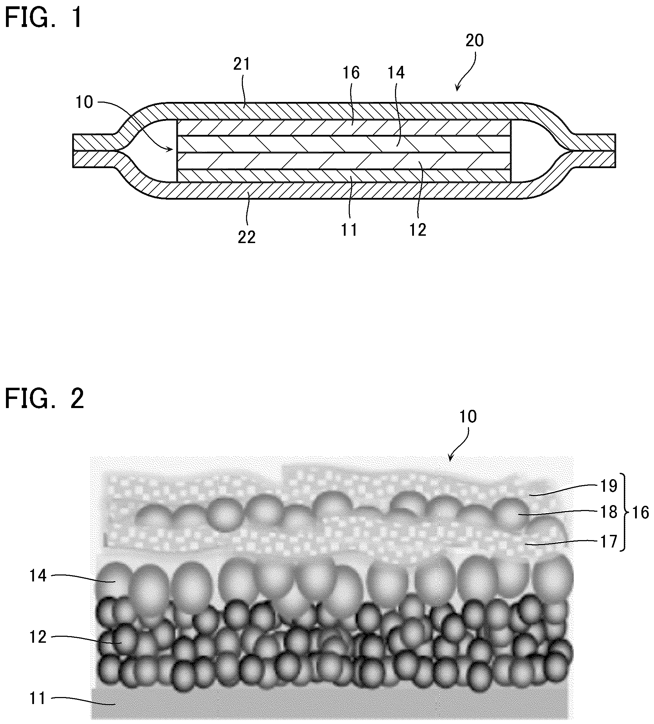

Hereinafter, embodiments according to the present invention will be described with reference to drawings. As illustrated in Fig. 1, a steam generator 20 according to the present embodiment includes a steam generating sheet 10, a first sheet 21 and a second sheet 22 that interpose and envelope the steam generating sheet 10 therebetween. As illustrated in Fig. 2, the steam generating sheet 10 includes a substrate layer 11, a heat generating layer 12 laminated on the substrate layer 11, a first water absorbent layer 14 laminated on the heat generating layer 12 and containing a first water absorbent, a water absorbing sheet 17 laminated on the first water absorbent layer 14, and a second water absorbent layer 18 laminated on the water absorbing sheet 17 and containing a second water absorbent.

The steam generating sheet 10 including the heat generating layer 12, the first water absorbent layer 14, the water absorbing sheet 17, and the second water absorbent layer 18 is heated by an oxidation reaction of an oxidizable metal contained in the heat generating layer 12 to generate a large amount of steam. Specifically, a maximum temperature of the steam generating sheet is 70°C or higher, and a cumulative steam generation amount during 10 minutes from the start of steam generation is 530 mg or more. In addition, a time required for the steam generating sheet to be heated from 35°C to 45°C is as short as 1.0 minute or shorter, and the temperature rises quickly.

Each of the layers of the steam generating sheet 10 will be described in detail below.

### (Substrate Layer)

A non-air permeable or hardly air permeable sheet can be used as the substrate layer 11. The hardly air permeability means that a degree of air permeability is 3,000 sec/100 mL or more, and the non-air permeability means that a degree of air permeability is 80,000 sec/100 mL or more. Note that the degree of air permeability is a value measured according to JIS P8117 (revised version in 2009) and is defined as a time for 100 mL of air to pass through an area of 6.42 cm² under a constant pressure.

A degree of air permeability of the substrate layer 11 is preferably 50,000 sec/100 mL or more, and more preferably 80,000 sec/100 mL or more. By using such a substrate layer 11, the generated steam is efficiently discharged from the second water absorbent layer 18 containing the second water absorbent. As the substrate layer 11, specifically, a synthetic resin film can be used, and examples of the substrate layer 11 can include a polyethylene film and a polyethylene terephthalate film. The synthetic resin film may be a single layer or a laminate having a plurality of layers. A nonwoven fabric, a paper, or a film, or a laminate of two or more thereof can be used.

### (Heat Generating Layer)

The heat generating layer 12 contains an oxidizable metal, a carbon component, and water. The heat generating layer 12 is a coated layer coated with a slurry-like heat generating composition containing these components. When the heat generating layer 12 is in contact with air, heat is generated by an oxidation reaction of the oxidizable metal, whereby water is heated and becomes steam. Thus, steam can be obtained by a simple method.

The oxidizable metal contained in the heat generating layer 12 is a metal that generates heat by the oxidation reaction, and can be selected from, for example, iron, aluminum, zinc, manganese, magnesium, and calcium. The oxidizable metal may be either a powder or fiber. Among them, an iron powder is preferable from the viewpoint of excellent handleability, safety, storage stability, and stability, and an inexpensive production cost. Examples of the iron powder can include a reduced iron powder and an atomized iron powder. The iron powders may be used alone or in combination of two or more thereof.

In a case where the oxidizable metal is a powder, an average particle size of the powder is preferably 10 µm or more, more preferably 20 µm or more, and still more preferably 25 µm or more. In addition, the average particle size is preferably 200 µm or less, more preferably 150 µm or less, and still more preferably 100 µm or less. The particle size of the oxidizable metal refers to a maximum length in a powdery form, and can be measured by classification with a sieve, a dynamic light scattering method, a laser diffraction and scattering method, or the like. By using the oxidizable metal having an average particle size in a predetermined range, an efficient oxidation reaction and a preferred coatability can be achieved.

A content of the oxidizable metal in the heat generating layer 12 is preferably 100 g/m² or more, more preferably 200 g/m² or more, and still more preferably 300 g/m² or more in terms of basis weight. In addition, the content of the oxidizable metal is preferably 3,000 g/m² or less, more preferably 2,000 g/m² or less, and still more preferably 1,500 g/m² or less. When a predetermined amount of the oxidizable metal is contained, the heat generating layer 12 can be heated to a desired temperature.

The content of the oxidizable metal can be determined by an ash test according to JIS P8128 or thermogravimetric instrument. In addition, the content can be quantified by a vibration sample type magnetization measurement test or the like by utilizing the property that generates magnetization when an external magnetic field is applied.

As the carbon component, a carbon component that can also act as a water absorbent and has water retainability, oxygen supply ability, and catalytic ability can be used. Examples of the carbon component can include activated carbon, acetylene black, and graphite. Activated carbon is preferable because oxygen is easily adsorbed at the time of being wetted, and moisture in the heat generating layer 12 can be kept constant. In addition, the activated carbon can easily control the amount of water supported on the heat generating layer within a specific range.

Among the activated carbons, at least one fine powdery material or small granular material selected from coconut shell carbon, wood charcoal, and peat coal is more preferable. Such activated carbons may be used alone or in combination of two or more thereof. Wood charcoal is most preferable from the viewpoint of easily maintaining the amount of water supported on the heat generating layer within a specific range and obtaining a preferred heating and humidifying effect.

An average particle size of the carbon component is preferably 10 µm or more, more preferably 12 µm or more, and still more preferably 15 µm or more. The average particle size is preferably 200 µm or less, more preferably 150 µm or less, and still more preferably 100 µm or less. By using a carbon component having an average particle size within a predetermined range, the carbon component can be uniformly mixed with the oxidizable metal. In addition, the amount of water supported on the heat generating layer 12 is easily maintained in a specific range.

The average particle size of the carbon component refers to a maximum length in a powdery form and is measured by a dynamic light scattering method, a laser diffraction method, or the like. The carbon component is preferably in a powdery form, but the form other than the powdery form, for example, a fibrous form can also be used.

As a fibrous carbon component, a natural or synthetic fibrous carbon component can be used, but is not particularly limited. Examples of a natural fibrous material can include plant fibers such as cotton, kapok, and wood pulp. In addition, examples of an animal fiber can include wool and goat hair. Furthermore, examples of a mineral fiber can include asbestos.

On the other hand, examples of a synthetic fibrous material can include synthetic polymer fibers such as rayon, polyester, polyethylene, and polyurethane. Furthermore, a metal fiber, a carbon fiber, a glass fiber, and the like may be used. These fibers can be used alone or as a mixture. Among them, at least one selected from wood pulp, cotton, a polyethylene fiber, and a polyester fiber is preferably used from the viewpoint of fixability with an oxidizable metal or a reaction accelerator, flexibility of the heat generating layer 12, oxygen permeability, a maintenance function of a sheet form, a production cost, and the like. In addition, wood pulp or cotton has a function of supporting and fixing a solid material such as an iron powder.

A content of the carbon component in the heat generating layer 12 is preferably 0.3 parts by mass or more, more preferably 1 part by mass or more, and still more preferably 3 parts by mass or more, with respect to 100 parts by mass of the oxidizable metal. In addition, the content of the carbon component in the heat generating layer 12 is preferably 20 parts by mass or less, more preferably 15 parts by mass or less, and still more preferably 13 parts by mass or less, with respect to 100 parts by mass of the oxidizable metal.

Note that the content of the carbon component in the heat generating layer 12 is preferably 4 g/m² or more, more preferably 7 g/m² or more, still more preferably 10 g/m² or more, and further still more preferably 15 g/m² or more in terms of basis weight. In addition, the content of the carbon component is preferably 290 g/m² or less, more preferably 200 g/m² or less, still more preferably 160 g/m² or less, and further still more preferably 120 g/m² or less in terms of basis weight.

The water contained in the heat generating layer 12 is not particularly limited. The water may be derived from an aqueous electrolyte solution (for example, an aqueous solution of an alkali metal, an alkaline earth metal, or the like) or may be water added to the heat generating layer 12 alone.

A content of the water in the heat generating layer 12 is preferably 6% by mass or more, more preferably 13% by mass or more, and still more preferably 15% by mass or more. When the content of the water is not less than a lower limit, steam is stably generated. In addition, the content of the water in the heat generating layer 12 is preferably 28% by mass or less, more preferably 27% by mass or less, and still more preferably 25% by mass or less. When the content of the water is not more than an upper limit, the rise of the temperature of the steam can be accelerated.

Note that the content of the water in the heat generating layer 12 can be determined by, for example, the following method. Specifically, about 1 g of the heat generating layer is collected, a mass thereof is precisely weighed, and then a mass after drying the collected heat generating layer is measured. As for drying conditions, for example, the drying can be performed at 150°C for 10 minutes. A mass difference before and after the drying can be divided by the mass of the collected heat generating layer to calculate the content of the water in the heat generating layer 12.

In the heat generating layer 12, a mass ratio of the content of the water to the content of the carbon component (water/carbon component) affects a speed of the temperature rise of the steam, the amount of steam generated, and the ease of controlling the temperature. In consideration of these conditions, the mass ratio of the content of the water to the content of the carbon component (water/carbon component) is preferably 0.5 or more, more preferably 0.55 or more, still more preferably 0.6 or more, and further still more preferably 1 or more. In addition, the mass ratio of the content of the water to the content of the carbon component (water/carbon component) is preferably 8.3 or less, more preferably 7.7 or less, still more preferably 7.0 or less, and further still more preferably 6.4 or less.

Furthermore, since the air permeability of the steam generating sheet 10 is sufficiently secured, an appropriate amount of oxygen is supplied to obtain the steam generating sheet 10 having a high heat generation effect. In addition, since the heat capacity of the steam generating sheet 10 with respect to a heating value to be obtained is suppressed to be small, the temperature rise is large. Therefore, a large amount of steam can be generated in a short time.

The heat generating layer 12 can further contain a reaction accelerator. When the reaction accelerator is contained, the oxidation reaction of the oxidizable metal is easily sustained. In addition, an oxide film formed by the oxidation reaction on the oxidizable metal is destructed to thereby accelerate the oxidation reaction. The reaction accelerator is selected from the group consisting of, for example, sulfate and chloride of an alkali metal or an alkaline earth metal, and the reaction accelerators may be used alone or in combination of two or more thereof. Among them, at least one selected from various chlorides such as sodium chloride, potassium chloride, calcium chloride, magnesium chloride, ferrous chloride, and ferric chloride, or sodium sulfate is preferable from the viewpoint of excellent conductivity and chemical stability and an inexpensive production cost.

When a content of the reaction accelerator in the heat generating layer 12 is 2 parts by mass or more with respect to the content of 100 parts by mass of the oxidizable metal, a desired effect can be obtained, and the content of the reaction accelerator is more preferably 3 parts by mass or more and still more preferably 4 parts by mass or more. In addition, the content of the reaction accelerator in the heat generating layer 12 is preferably 15 parts by mass or less, more preferably 13 parts by mass or less, and still more preferably 11 parts by mass or less, with respect to the content of 100 parts by mass of the oxidizable metal.

The heat generating layer 12 can further contain a thickener. As the thickener, a material that absorbs moisture to increase the consistency or a material that imparts thixotropic properties can be used. Specific examples of the thickener can include a polysaccharide-based thickener such as alginate such as sodium alginate, gum arabic, gum tragacanth, locust bean gum, guar gum, gum arabic, carrageenan, agar, or xanthan gum; a starch-based thickener such as dextrin, α starch, or processing starch; a cellulose derivative-based thickener such as carboxymethyl cellulose, ethyl cellulose acetate, hydroxyethyl cellulose, hydroxymethyl cellulose, or hydroxypropyl cellulose; a metallic soap-based thickener such as stearate; and a mineral-based thickener such as bentonite. These thickeners may be used alone or in combination of two or more thereof.

Among them, a polysaccharide-based thickener is preferable from the viewpoint that a preferred coatability or the amount of water supported on the heat generating layer can be maintained in a specific range. Furthermore, a polysaccharide-based thickener having a molecular weight of 1,000,000 or more and 50,000,000 or less is preferable, a polysaccharide-based thickener having a molecular weight of 1,500,000 or more and 45,000,000 or less is more preferable, and polysaccharide-based thickener having a molecular weight of 2,000,000 or more and 40,000,000 or less is still more preferable. In addition, xanthan gum is preferable from the viewpoint of having a preferred coatability or salt resistance.

A content of the thickener in the heat generating layer 12 is preferably 0.05 parts by mass or more, more preferably 0.1 parts by mass or more, and still more preferably 0.12 parts by mass or more, with respect to 100 parts by mass of the oxidizable metal. In addition, the content of the thickener in the heat generating layer 12 is preferably 5 parts by mass or less, more preferably 4 parts by mass or less, and still more preferably 3.5 parts by mass or less, with respect to 100 parts by mass of the oxidizable metal.

When the thickener is contained in an amount within a predetermined range, solid contents such as the oxidizable metal and the carbon component can be stably dispersed. In addition, the thixotropic properties can be imparted to the heat generating composition, and the coatability can be further improved. Furthermore, the amount of water supported on the heat generating layer 12 can be easily maintained within a predetermined range.

The heat generating layer 12 can contain a surfactant, a medicine, a flocculant, a coloring agent, a paper strengthening agent, a pH adjusting agent (for example, tripotassium phosphate or the like), and a bulking agent, if necessary.

The heat generating layer 12 can be produced by applying a slurry-like heat generating composition prepared by blending a predetermined component on a surface of the substrate layer 11 to form a composition layer and then spraying sodium chloride or the like as a reaction accelerator.

A thickness of the heat generating layer 12 is preferably 0.2 mm or more, more preferably 0.4 mm or more, and still more preferably 0.5 mm or more. On the other hand, the thickness of the heat generating layer 12 is preferably 10 mm or less, more preferably 6 mm or less, and still more preferably 4 mm or less. When the thickness of the heat generating layer 12 is within the above range, the heat generating layer 120 can be easily sized while enhancing the heat generation effect. Note that the thickness of the heat generating layer 12 indicates an average thickness.

### (First Water Absorbent Layer Containing First Water Absorbent)

The first water absorbent layer 14 containing a first water absorbent is a layer formed by spraying the first water absorbent onto the heat generating layer 12. As described above, since the first water absorbent layer 14 is directly laminated on the heat generating layer 12, excessive water in the heat generating layer 12 can be efficiently absorbed and maintained at an appropriate water amount.

In the present specification, the water absorbent has water retainability (water absorbing action or water removing action), and is selected from, for example, a carbon component, a fiber material, and a water absorbing powder. The water absorbents can be used alone or in combination of two or more thereof.

Note that in the present invention "laminated" does not need to be a completely separated individual layer. For example, a part of the first water absorbent constituting the first water absorbent layer 14 may be mixed in the heat generating layer 12.

As the carbon component, for example, one or two or more selected from activated carbon, acetylene black, and graphite can be used. Such a component has oxygen supply ability and catalytic ability in addition to water retainability. Activated carbon is preferable because moisture in the heat generating layer can be kept constant. Specific examples of the carbon component can include fine powdery material or small granular coconut shell carbon, wood charcoal, and peat coal, and wood charcoal is preferable.

As the fiber material, a hydrophilic fiber, particularly, a cellulose fiber is more preferable. As the cellulose fiber, a chemical fiber (synthetic fiber) or a natural fiber can be used.

Examples of the water absorbing powder can include one or two or more selected from vermiculite, sawdust, silica gel, a pulp powder, and a water absorbing polymer.

Examples of the water absorbing polymer can include a hydrophilic polymer having a crosslinked structure capable of absorbing and holding a liquid not less than 20 times of its own weight. A shape of the water absorbing polymer may be any one of a spherical shape, a massive shape, a grape bunch shape, and a fibrous shape. Hereinafter, the water absorbing polymer will be described as an example of the water absorbent.

A mass average particle size of the water absorbing polymer used for formation of the first water absorbent layer 14 (hereinafter, referred to as a first water absorbing polymer) is preferably 1 µm or more, more preferably 10 µm or more, and still more preferably 100 µm or more. In addition, the mass average particle size of the first water absorbing polymer is preferably 1,000 µm or less, more preferably 700 µm or less, and still more preferably 500 µm or less. The particle size of the first water absorbing polymer is measured by a dynamic light scattering method, a laser diffraction method, or the like.

Specific examples of the first water absorbing polymer can include one or two or more selected from the group consisting of starch, crosslinked carboxyl methylated cellulose, a polyacrylic acid and a salt thereof, and a polyacrylate graft polymer such as a polymer or copolymer of an acrylic acid or an alkali metal salt of an acrylic acid. Among them, a polyacrylic acid and a salt thereof, and a polyacrylate graft polymer such as a polymer or copolymer of an acrylic acid or an alkali metal salt of an acrylic acid are preferable from the viewpoint that the amount of water supported is maintained within a specific range.

A basis weight of the first water absorbing polymer contained in the first water absorbent layer 14 is preferably 15 g/m² or more, more preferably 20 g/m² or more, and still more preferably 25 g/m² or more in a dry state from the viewpoint of a speed of the temperature rise of the steam. In addition, an upper limit of the basis weight of the first water absorbing polymer contained in the first water absorbent layer 14 is not particularly limited, but is preferably 300 g/m² or less, more preferably 200 g/m² or less, and still more preferably 160 g/m² or less in the dry state from the viewpoint of cost-effectiveness.

The first water absorbent layer 14 can be produced by directly spraying the first water absorbing polymer onto a surface of the heat generating layer 12.

### (Water Absorbing Sheet Laminated on First Water Absorbent Layer and Second Water Absorbent Layer Laminated on Water Absorbing Sheet and Containing Second Water Absorbent)

On the first water absorbent layer 14, a first water absorbing sheet 17 and the second water absorbent layer 18 laminated on the first water absorbing sheet 17 and containing the second water absorbent are provided, and a second water absorbing sheet 19 laminated on the second water absorbent layer 18 is more preferably provided. A layer including the first water absorbing sheet 17 and the second water absorbent layer 18 is also referred to as the water-retention layer 16. Examples of the second water absorbent can include the carbon component as described above. Hereinafter, the water absorbing polymer will be described as an example.

The water-retention layer 16 sucks up water from the heat generating layer 12 and the first water absorbent layer 14. Therefore, the temperature of the heat generating layer is not lowered by the excessive water. As a result, the steam generating sheet has a higher maximum temperature than that of the related art, and can generate a large amount of steam. That is, in the steam generating sheet 10 of the present invention, the first water absorbent layer 14 is directly laminated on the heat generating layer 12, such that a part of the first water absorbent layer 14 is in contact with the heat generating layer 12, and the second water absorbent layer 18 is laminated with the first water absorbing sheet 17 interposed between the heat generating layer 12 and the second water absorbent layer 18.

When a steam generating sheet is provided with such a second water absorbent layer 18, and in which the water-retention layer 16 including the second water absorbent layer 18 sucks up water from the heat generating layer 12 and the first water absorbent layer 14, the steam generating sheet is within the scope of the present invention, and the laminated structure and the production method are not limited at all.

The first water absorbing sheet 17 is preferably a hydrophilic fiber sheet, particularly, a cellulose fiber sheet. As the cellulose fiber, either a chemical fiber (synthetic fiber) or a natural fiber may be used. A basis weight thereof is preferably 5 g/m² or more, and more preferably 10 g/m² or more, and still more preferably 12 g/m² or more. In addition, the basis weight is preferably 100 g/m² or less, more preferably 60 g/m² or less, and still more preferably 50 g/m² or less.

As long as the effect of the present invention is not impaired, a part of the first water absorbent constituting the first water absorbent layer 14 may be contained in the first water absorbing sheet 17. In addition, as long as the effect of the present invention is not impaired, a part of the second water absorbent constituting the second water absorbent layer 18 may also be contained in the first water absorbing sheet.

As the water absorbing powder used for formation of the second water absorbent layer 18, one or two or more selected from vermiculite, sawdust, silica gel, and a pulp powder may be used.

As the water absorbing polymer used for formation of the second water absorbent layer 18 (hereinafter, referred to as a second water absorbing polymer), the same water absorbing polymer as the first water absorbing polymer can be used. A basis weight of the second water absorbing polymer can be determined in consideration of the basis weight of the water absorbing sheet as described later.

As the second water absorbing sheet 19, a sheet formed of the same material as that of the first water absorbing sheet 17 can be used. A basis weight thereof is preferably 5 g/m² or more, and more preferably 10 g/m² or more, and still more preferably 12 g/m² or more. In addition, the basis weight is preferably 400 g/m² or less, more preferably 300 g/m² or less, and still more preferably 250 g/m² or less.

For example, when a total basis weight of the first water absorbing sheet 17 and the second water absorbing sheet 19 is about 50 g/m², the basis weight of the second water absorbing polymer is preferably 2 g/m² or more, more preferably 4 g/m² or more, and still more preferably 8 g/m² or more in a dry state. An upper limit of the basis weight of the second water absorbing polymer in the water-retention layer 16 is not particularly limited.
The basis weight of the second water absorbing polymer is preferably 200 g/m² or less, more preferably 150 g/m² or less, and still more preferably 100 g/m² or less in the dry state from the viewpoint of cost-effectiveness.

For example, when the total basis weight of the first water absorbing sheet 17 and the second water absorbing sheet 19 is 60 g/m² or more, the basis weight of the second water absorbing polymer is preferably 1 g/m² or more, more preferably 3 g/m² or more, and still more preferably 5 g/m² or more in the dry state. An upper limit of the basis weight of the second water absorbing polymer in the water-retention layer 16 is not particularly limited. The basis weight of the second water absorbing polymer is preferably 150 g/m² or less, more preferably 120 g/m² or less, and still more preferably 90 g/m² or less in the dry state from the viewpoint of cost-effectiveness.

In addition, for example, when the total basis weight of the first water absorbing sheet 17 and the second water absorbing sheet 19 is 40 g/m² or less, the basis weight of the second water absorbing polymer is preferably 5 g/m² or more, more preferably 10 g/m² or more, and still more preferably 15 g/m² or more in the dry state. An upper limit of the basis weight of the second water absorbing polymer in the water-retention layer 16 is not particularly limited. The basis weight of the second water absorbing polymer is preferably 250 g/m² or less, more preferably 200 g/m² or less, and still more preferably 150 g/m² or less in the dry state from the viewpoint of cost-effectiveness.

In the present embodiment, the water-retention layer 16 can be produced by spraying the second water absorbing polymer onto a surface of the first water absorbing sheet 17 and forming the second water absorbent layer 18. Furthermore, in a case where the second water absorbing sheet 19 is included in the water-retention layer 16, the second water absorbing sheet 19 is further laminated on the second water absorbent layer 18.

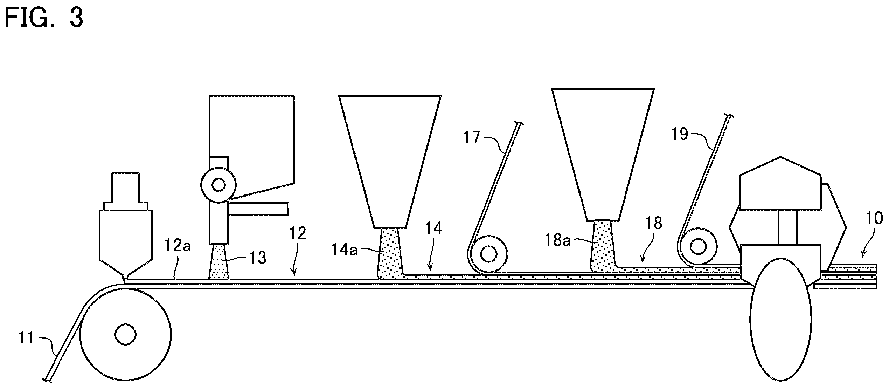

The steam generating sheet 10 of the present embodiment can be produced in-line, for example, as illustrated in Fig. 3. First, a slurry-like heat generating composition is applied to the surface of the substrate layer 11 to form a composition layer 12a. The heat generating layer 12 is obtained by spraying a reaction accelerator 13 such as sodium chloride or the like onto the composition layer 12a.

In a case where the heat generating layer 12 is produced by application, an application method is not particularly limited, but for example, die coating, roll coating, screen printing, roll gravure, knife coating, curtain coater, or the like can be used.

The first water absorbent layer 14 is formed by spraying particles 14a of the first water absorbing polymer onto the heat generating layer 12. Next, the first water absorbing sheet 17 is laminated and particles 18a of the second water absorbing polymer are sprayed onto the first water absorbing sheet 17 to form the second water absorbent layer 18, and more preferably, the second water absorbing sheet 19 is laminated on the second water absorbent layer 18 to be integrated with the second water absorbent layer 18. The steam generating sheet 10 is produced by cutting the obtained laminate.

It is preferable to obtain the steam generating sheet 10 by supplying a polymer sheet produced in advance as the water-retention layer 16 onto the first water absorbent layer 14 from the viewpoint of improving productivity and heat generation characteristics. The polymer sheet can be produced by integrating the first water absorbing sheet 17, the second water absorbent layer 18, and the second water absorbing sheet 19.

As described above, in the present invention, a desired generator can be obtained by providing water absorbent layers via the water absorbing sheet.

The first sheet 21 is disposed on the water-retention layer 16 side of the steam generating sheet 10, the second sheet 22 is disposed on the substrate layer 11 side, and peripheries of the two sheets are sealed and joined to obtain the steam generator 20 of the present embodiment as illustrated in Fig. 1.

A degree of air permeability of the first sheet 21 is preferably 300 sec/100 mL or less, more preferably 200 sec/100 mL or less, still more preferably 100 sec/100 mL or less, further still more preferably 5 sec/100 mL or less, and further still more preferably 0 sec/100 mL. When such a degree of air permeability is satisfied, the first sheet 21 may be a non-air permeable sheet with a part having no air permeability. By using the first sheet 21 having a degree of air permeability of 100 sec/100 mL or less, steam from the steam generating sheet 10 can be efficiently discharged from the first sheet 21.

A thickness of the first sheet 21 is preferably 5 µm or more, more preferably 10 µm or more, and still more preferably 20 µm or more, and is preferably 500 µm or less, more preferably 450 µm or less, and still more preferably 400 µm or less.

As the first sheet 21, for example, a porous sheet formed of a synthetic resin having moisture permeability and having no water permeability is suitable. Specific examples thereof can include a film obtained by adding calcium carbonate or the like to polyethylene or polypropylene and performing stretching. In a case where such a porous sheet is used, a nonwoven fabric may be laminated on an outer surface of the porous sheet to enhance the texture of the first sheet 21. Examples of the nonwoven fabric can include a needle punch nonwoven fabric, an air-through nonwoven fabric, and a spunbonded nonwoven fabric.

When the second sheet 22 is a sheet having low air permeability as a whole, a part thereof may have air permeability. Specifically, a degree of air permeability of the second sheet 22 is preferably 8,000 sec/100 mL or more, more preferably more than 8,000 sec/100 mL, still more preferably 20,000 mL or more, and further still more preferably 50,000 sec/100 mL or more, that is, the second sheet 22 is more preferably a non-air permeable sheet. As a result, steam can be efficiently discharged from the first sheet 21. Specifically, the second sheet 22 can be formed by laminating a single synthetic resin film or a plurality of synthetic resin films. Note that the degree of air permeability is preferably 100,000 sec/100 mL or less from the viewpoint of availability.

A thickness of the second sheet 22 is preferably 5 µm or more, more preferably 10 µm or more, and still more preferably 20 µm or more, and is preferably 500 µm or less, more preferably 450 µm or less, and still more preferably 400 µm or less.

As long as the degree of air permeability is satisfied, depending on the application, a nonwoven fabric or the like may be laminated on an outer surface of the second sheet 22 to enhance the texture of the second sheet 22. The nonwoven fabric can be selected from a needle punch nonwoven fabric, an air-through nonwoven fabric, and a spunbonded nonwoven fabric, but a laminate film formed of a polyethylene film and a pulp sheet is preferable.

As long as the value of the degree of air permeability is satisfied, the second sheet 22 may be formed of the same material as that of the first sheet 21 or may be formed of a material different from that of the first sheet 21.

### <Heating Tool>

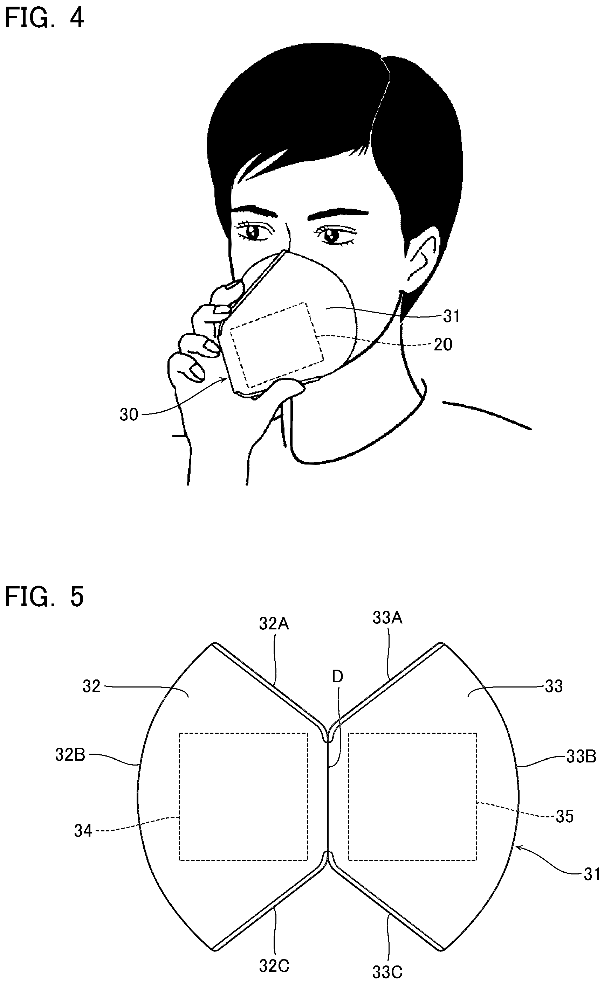

Next, the heating tool including the steam generator 20 of the present embodiment will be described. Since the steam generator of the present invention can generate a large amount of steam in a short time, it is particularly preferable to use a heating tool that supplies steam to a mouth and a nose. For example, as illustrated in Fig. 4, a heating tool 30 includes a main body portion 31 that covers a mouth and a nose of a user and the steam generator 20 provided in the main body portion 31. The main body portion 31 of the heating tool 30 has rigidity enough to retain the shape when held by the hand of the user, and can be used in a state where the main body portion 31 is held by the hand and the mouth and the nose of the user are covered with the main body portion 31.

Note that, although Fig. 4 illustrates the heating tool to be used with a hand, the present invention is not limited thereto, and a mask having an ear hook may be used.

As illustrated in Fig. 5, the main body portion 31 includes one sheet in which tapered ends of a first panel 32 having a substantially fan shape and a second panel 33 having the same shape as that of the first panel 32 are continuous. A boundary line D between the first panel 32 and the second panel 33 is bent to overlap the first panel 32 and the second panel 33 each other, and a side edge 32A of the first panel 32 and a side edge 33A of the second panel 33 and a side edge 32C of the first panel 32 and a side edge 33C of the second panel 33 are joined to each other, respectively. The main body portion 31 is formed in a bag shape having a tapered shape tapered toward a bottom side with the boundary line D as the bottom side and the side edge 32B and the side edge 33B as an opening. The opening of the main body portion 31 is opened to the extent that the nose and the mouth of the user can be covered.

The first panel 32 and the second panel 33 can be produced by cutting a sheet in which two types of nonwoven fabrics having different basis weights and formed of different materials are laminated into a predetermined shape. The first panel 32 and the second panel 33 are provided with storage portions 34 and 35 that can store and fix the steam generator 20, respectively, between the two nonwoven fabrics.

Note that, as a fiber material of the nonwoven fabric used for the first panel 32 and the second panel 33, it is preferable to use a material formed of one or two or more fibers selected from polyester such as polyethylene terephthalate (PET); polyolefin such as polyethylene (PE), polypropylene (PP), or an ethylene propylene copolymer; rayon; and cotton. In addition, as the nonwoven fabric, a nonwoven fabric produced by an air-through method, a spunbond method, a needle punch method, a melt blown method, a card method, a thermal fusion method, a water flow entangling method, a solvent adhesion method, or the like using the fiber formed of the one or two or more materials can be used.

The heating tool 30 may be perfumed with a perfume composition. In this case, the perfume composition can be directly added, for example, by being sprayed onto at least one of the main body portion 31 of the heating tool 30 and the steam generator 20. Alternatively, perfume may be imparted to the raw material of the first sheet 21, the second sheet 22, or the steam generating sheet 10 in the steam generator 20.

In addition, the steam generator 20 of the present invention may be attached to a three-dimensional shaped vessel such as a paper cup to form a heating tool.

Here, the heating tool (steam cup) illustrated in Fig. 4 will be described.

The heating tool is characterized in that the heating tool does not have an ear hook and a user can freely adjust the amount of steam by holding the heating tool in a hand at the time of use. In the case of such a steam cup, the main body portion 31 preferably has appropriate air permeation resistance from the viewpoint of retaining the steam generated from the steam generator 20 in the heating tool 30 and from the viewpoint of making breathing easy.

Specifically, the air permeation resistance of the main body portion 31 is preferably 5 Pa or more, more preferably 20 Pa or more, and still more preferably 50 Pa or more. In addition, the air permeation resistance of the main body portion 31 is preferably 300 Pa or less, more preferably 250 Pa or less, and still more preferably 200 Pa or less.

In addition, the air permeation resistance of the main body portion 31 is preferably 5 Pa or more and 300 Pa or less, more preferably 20 Pa or more and 250 Pa or less, and still more preferably 50 Pa or more and 200 Pa or less. Note that when the structure of the main body portion 31 is multiplied, the air permeation resistance is an air permeation resistance measured in a state where all of the plurality of sheets are overlapped.

The air permeation resistance of the main body portion 31 can be measured as follows.

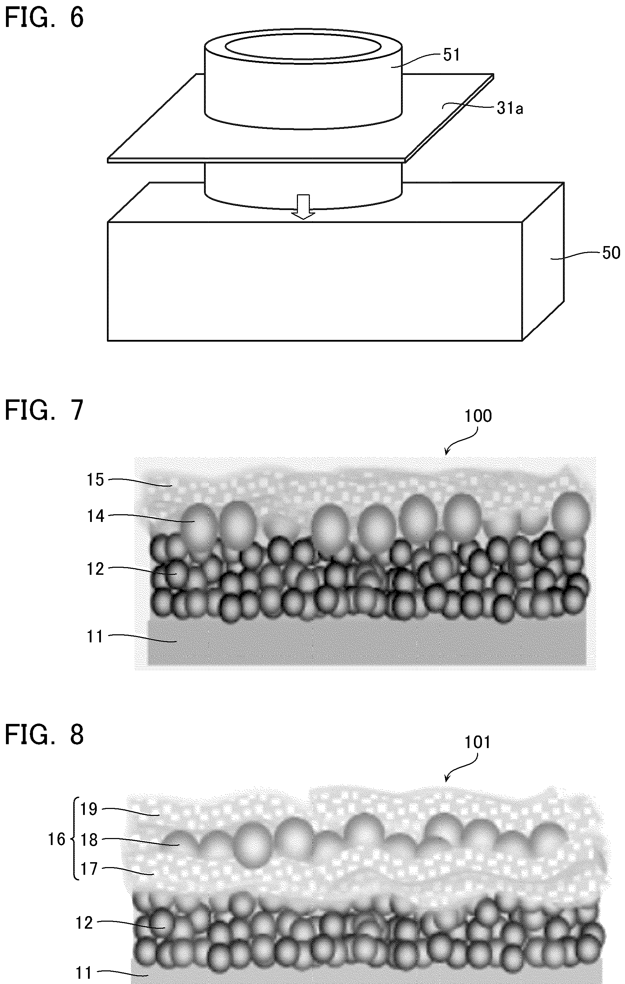

As illustrated in Fig. 6, a sheet 31a cut into a size of 3.5 to 5 cm square from the sheet material of the main body portion 31 is disposed on an upper portion of a main body 50 of an air permeation resistance evaluation device of mask tester MTS-2 (manufactured by Shibata Scientific Technology Ltd.) and the sheet 31a is fixed with a sheet fixing tool 51 so as not to leak. The measurement is performed at a test area of 7 cm² (inner diameter: 30 mm) and a test flow rate of 10 L/min for 10 seconds, and the air permeation resistance is obtained from a differential pressure between an air inflow side (inlet side) and an air outflow side (outlet side) of the sheet 31a.

A basis weight of the main body portion 31 is preferably 5 g/m² or more, more preferably 10 g/m² or more, still more preferably 30 g/m² or more, and is preferably 250 g/m² or less, more preferably 200 g/m² or less, and still more preferably 150 g/m² or less, from the viewpoint of enhancing heat retention, thickness, and sheet strength with good balance.

In addition, the basis weight of the main body portion 31 is preferably 5 g/m² or more and 250 g/m² or less, more preferably 10 g/m² or more and 200 g/m² or less, and still more preferably 30 g/m² or more and 150 g/m² or less.

The above-described steam cup can be held by a hand of a user to freely adjust the amount of steam, whereas a mask (steam mask) having an ear hook does not need to be held by a hand at the time of use and is also used to obtain a constant amount of steam.

In the case of such a steam mask, the air permeation resistance of the main body portion 31 is preferably 5 Pa or more, more preferably 20 Pa or more, and still more preferably 50 Pa or more. In addition, the air permeation resistance of the main body portion 31 is preferably 200 Pa or less, more preferably 190 Pa or less, and still more preferably 180 Pa or less.

In addition, the air permeation resistance of the main body portion 31 is preferably 5 Pa or more and 200 Pa or less, more preferably 20 Pa or more and 190 Pa or less, and still more preferably 50 Pa or more and 180 Pa or less.

In the case of the steam mask, the basis weight of the main body portion 31 is preferably 5 g/m² or more, more preferably 10 g/m² or more, and still more preferably 30 g/m² or more, and is preferably 200 g/m² or less, more preferably 150 g/m² or less, and still more preferably 120 g/m² or less.

In addition, the basis weight of the main body portion 31 is preferably 5 g/m² or more and 200 g/m² or less, more preferably 10 g/m² or more and 150 g/m² or less, and still more preferably 30 g/m² or more and 120 g/m² or less.

### Examples

Next, examples of the present invention will be described, but the present invention is not limited thereto.

### <Production of Steam Generating Sheet>

A heat generating composition having the composition shown in Table 1 was prepared by the following procedure. First, xanthan gum as a thickener was dissolved in water, and then tripotassium phosphate was dissolved to prepare an aqueous solution. On the other hand, an iron powder, activated carbon, potassium hydroxide, and water were pre-mixed with each other to prepare a slurry. The pre-mixed slurry was added to the aqueous solution, stirring was performed with disk turbine type stirring blades at 150 rpm for 10 minutes, thereby obtaining a slurry-like heat generating composition.

**[Table 1]**

| Component | | Manufacturer | Parts by mass |
|---|---|---|---|
| Iron powder (average particle size: 45 µm) | Reduced iron powder | DOWA IP CREATION Co., Ltd. | 100 |
| Water | Tap water | | 62 |
| Activated carbon | Carboraffin | Osaka Gas Chemicals Co., Ltd. | 8 |
| Xanthan gum | Rhaball gum GS-C | DSP GOKYO FOOD & CHEMICAL Co., Ltd. | 0.2 |
| Tripotassium phosphate | | YONEYAMA CHEMICAL INDUSTRY CO., LTD. | 1.8 |
| Potassium hydroxide | Potassium hydroxide solution (48%) | AGC Inc. | 0.2 |
| Total | | | 172.2 |

A polyethylene laminate crepe paper (basis weight: 97 g/m², manufactured by Daishowa Paper Products Co., Ltd.) was prepared as the substrate layer 11. The heat generating composition was applied to a region of 24.0 cm² (4.9 cm × 4.9 cm) of a pulp surface of the substrate layer 11 to form a composition layer. The mass of the heat generating composition used was 4.6 g. 0.171 g of a salt (sodium chloride designated by Japanese Pharmacopoeia (manufactured by Otsuka Pharmaceutical Co., Ltd.)) was sprayed onto the surface (24.0 cm²) of the composition layer to form a heat generating layer 12. Using the substrate layer 11 on which the heat generating layer 12 was formed, steam generating sheets (Nos. 1 to 19) having different configurations were produced as follows.

### (Nos. 1 to 4)

A steam generating sheet 100 having the configuration illustrated in Fig. 7 was produced. The steam generating sheet 100 is provided with no second water absorbent layer 18. In the production, first, a water absorbing polymer (sodium polyacrylate, spherical, average particle size: 300 µm, SUNFRESH ST-500D*, manufactured by SANYO CHEMICAL INDUSTRIES, LTD.) was directly sprayed (the basis weight of each production example was as shown in Table 2) onto the heat generating layer 12 to form a first water absorbent layer 14 containing a first water absorbent on the heat generating layer 12. A kraft paper 15 (basis weight: 50 g/m², manufactured by Daishowa Paper Products Co., Ltd.) as a water absorbing sheet was laminated on the first water absorbent layer 14 and integrated to prepare each of the steam generating sheets Nos. 1 to 4.

### (Nos. 5 to 8)

A steam generating sheet 101 having the configuration illustrated in Fig. 8 was produced. The steam generating sheet 101 is provided with no first water absorbent layer 14. In the production, a polymer sheet (PS) as a water-retention layer 16 was directly laminated on the heat generating layer 12. As the polymer sheet, a wood pulp paper (basis weight: 30 g/m², manufactured by Inokami Co., Ltd.) as a first water absorbing sheet 17, a second water absorbent layer 18 (the basis weight of each production example was as shown in Table 2) containing a second water absorbing polymer (sodium polyacrylate, spherical, average particle size: 300 µm, AQUALIC CA, manufactured by Nippon Shokubai Co., Ltd.), and a wood pulp paper (basis weight: 20 g/m², manufactured by Inokami Co., Ltd.) as a second water absorbing sheet 19 were laminated and integrated. The polymer sheet was laminated so that the first water absorbing sheet 17 was in contact with the heat generating layer 12.

### (Nos. 9 and 10)

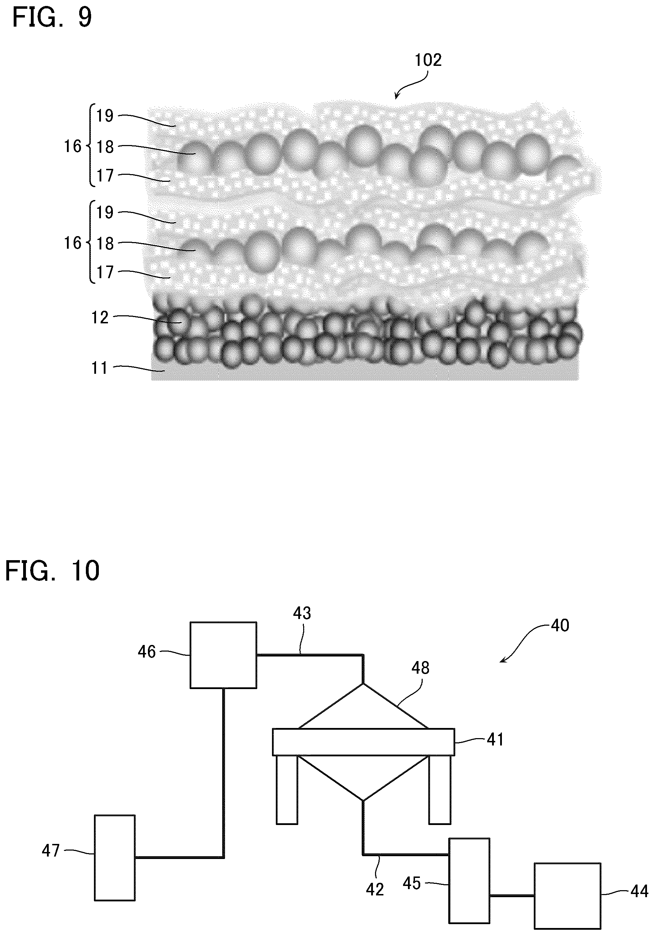

A steam generating sheet 102 having the configuration illustrated in Fig. 9 was produced. The steam generating sheet 102 is provided with no first water absorbent layer 14. In the production, two of polymer sheet as a water-retention layer 16 were laminated on the heat generating layer 12.

As the polymer sheet as the water-retention layer 16, a wood pulp paper (basis weight: 30 g/m², manufactured by Inokami Co., Ltd.) as a first water absorbing sheet 17, a second water absorbent layer 18 (the basis weight of each production example was as shown in Table 2) containing a second water absorbing polymer (sodium polyacrylate, spherical, AQUALIC CA, manufactured by Nippon Shokubai Co., Ltd.), and a wood pulp paper (basis weight: 20 g/m², manufactured by Inokami Co., Ltd.) as a second water absorbing sheet 19 were laminated and integrated. First polymer sheet was laminated so that the first water absorbing sheet 17 was in contact with the heat generating layer 12 and first water absorbing sheets 17 of second polymer sheet was in contact with second water absorbing sheet 19 of the first polymer sheet.

### (Nos. 11 to 19)

A steam generating sheet 10 having the configuration illustrated in Fig. 2 was produced. A first water absorbing polymer (sodium polyacrylate, spherical, average particle size: 300 µm, SUNFRESH ST-500D*, manufactured by SANYO CHEMICAL INDUSTRIES, LTD.) was directly sprayed (the basis weight of each production example was as shown in Table 2) onto the heat generating layer 12 to form a first water absorbent layer 14. The following polymer sheet as a water-retention layer 16 was laminated on the first water absorbent layer 14 to produce the steam generating sheets Nos. 11 to 19.

As the polymer sheet as the water-retention layer 16, a wood pulp paper (basis weight: 30 g/m², manufactured by Inokami Co., Ltd.) as a first water absorbing sheet 17, a second water absorbent layer 18 (the basis weight of each production example was as shown in Table 2) containing a second water absorbing polymer (sodium polyacrylate, AQUALIC CA, manufactured by Nippon Shokubai Co., Ltd.), and a wood pulp paper (basis weight: 20 g/m², manufactured by Inokami Co., Ltd.) as a second water absorbing sheet 19 were laminated and integrated. The polymer sheet was laminated so that the first water absorbing sheet 17 was in contact with the first water absorbent layer 14.

In the obtained steam generating sheet, as illustrated in Fig. 1, the first sheet 21 and the second sheet 22 were placed up and down, and peripheries of the two seals were sealed to obtain steam generators. As described above, the second sheet 22 was disposed on a lower side (on the substrate layer 11) of the steam generating sheet.

Note that the first sheet 21 used here is "BRN-502" (the degree of air permeability is 100 sec/100 mL or less) manufactured by Nitoms, Inc., and the second sheet 22 is "LF-KIPE71" (non-air permeable, basis weight: 71 g/m²) manufactured by Kakukei Co., Ltd.

In addition, the area of the first sheet including an air permeable surface and a seal portion was 39.7 cm² (6.3 cm × 6.3 cm). This is defined as one sheet. Each of the steam generators was placed and stored in an oxygen blocking bag until evaluation described later was performed.

### <Production of Prototype of Heating Tool>

A first nonwoven fabric (needle punch (polypropylene) basis weight: 80 g/m²) and a second nonwoven fabric (spunbond (polypropylene) basis weight: 90 g/m²) were laminated to produce an exterior bag (7.5 cm × 7.5 cm). The steam generator was stored in the exterior bag so that the second sheet 22 was disposed on the side of the second nonwoven fabric and the periphery was sealed to obtain a prototype of a heating tool. The prototype of the heating tool was placed and stored in an oxygen blocking bag until evaluation described later was performed.

The amount of steam generated in the steam generator was measured, and heat generation characteristics of the heating prototype were examined. These measurement methods will be described below.

### <Amount of Steam Generated>

The amount of steam generated was measured by the following method using a measuring device 40 illustrated in Fig. 10.

The measuring device 40 includes an aluminum measurement chamber (volume: 4.2 L) 41, an inflow path 42 through which dehumidified air (humidity: less than 2%, flow rate: 2.1 L/min) flows into a lower portion of the measurement chamber 41, an outflow path 43 through which air flows out from an upper portion of the measurement chamber 41, an inlet temperature and humidity meter 44 and an inlet flow rate meter 45 provided in the inflow path 42, an outlet temperature and humidity meter 46 and an outlet flow rate meter 47 provided in the outflow path 43, and a thermometer (thermistor) 48 provided in the measurement chamber 41. As the thermometer 48, a thermometer having a temperature resolution of approximately 0.01°C was used.

The steam generator was taken out from the oxygen blocking bag at a measurement environmental temperature of 30°C (30 ± 1°C) and the steam generator was placed in the measurement chamber 41 with the surface (steam discharging surface) located on a skin side facing upward. The thermometer 48 with a metal ball (4.5 g) was placed on the steam discharging surface, and the temperature of the steam discharging surface was measured.

In addition, in this state, dehumidified air flowed from the lower portion of the measurement chamber 41, and measurement was performed with the inlet temperature and humidity meter 44 and the outlet temperature and humidity meter 46. From the obtained temperature and humidity, a difference in absolute humidity before and after air flows into the measurement chamber 41 was obtained. Further, the amount of steam discharged from the steam generator was calculated from the flow rates measured by the inlet flow rate meter 45 and the outlet flow rate meter 47.

Note that the amount of steam generated refers to a total amount of steam measured up to 10 minutes after the time when the steam generator is taken out from the oxygen blocking bag as a starting point. In order to sufficiently wet a throat and a nose, the amount of steam generated may be 530 mg or more, and may be preferably 535 mg or more.

### <Heat Generation Characteristics>

Using a measuring machine according to JIS S4100, the second nonwoven fabric of the prototype of the heating tool was attached to the measurement surface, and heat generation measurement was performed. In the heat generation measurement, a time axis was plotted on a horizontal axis, and the temperature was plotted on a vertical axis.

The maximum temperature and temperature rise time were determined from the plotted results. Specifically, the maximum temperature was the highest temperature (°C) from the start of the measurement to the end of the measurement, and the temperature rise time was a time (minute) required to reach from 35°C to 45°C from the start of the measurement. The higher the maximum temperature and the shorter the temperature rise time, the better the heat generation characteristics of the steam generator. The heat generation characteristics of the prototype of the heating tool correspond to the heat generation characteristics of the steam generator. The maximum temperature may be 70°C or higher, and may be preferably 71°C or higher, from the viewpoint of efficiently generating steam.

In addition, the time (minute) required to reach from 35°C to 45°C may be 1.0 minute or shorter and may be preferably 0.9 minutes or shorter so that the heating tool can be used immediately when desired.

A heating tool satisfying the amount of steam generated and the heat generation characteristics was regarded as pass.

The obtained results are summarized in Tables 2 and 3 together with the configuration of the steam generating sheet used.

**[Table 2]**

| Condition | | 1 | 2 | 3 | 4 | 5 | 6 | 7 | 8 | 9 | 10 |
|---|---|---|---|---|---|---|---|---|---|---|---|
| Configuration | First water absorbent layer | Present | Present | Present | Present | - | - | - | - | - | - |
| | Water absorbing sheet | Present | Present | Present | Present | Present | Present | Present | Present | Present | Present |
| | Second water absorbent layer | - | - | - | - | Present | Present | Present | Present | Present | Present |
| Spray amount of first water absorbing polymer | Basis weight (g/m²) | 80 | 100 | 120 | 140 | - | - | - | - | - | - |
| | Weight (g) | 0.192 | 0.240 | 0.288 | 0.336 | - | - | - | - | - | - |
| PS^{*2} | Basis weight (g/m2)^{*1} | - | - | - | - | 130 | 150 | 170 | 190 | 100 | 120 |
| | Weight (g) | - | - | - | - | 0.312 | 0.360 | 0.408 | 0.456 | 0.24 | 0.288 |
| PS^{*3} on heat generating layer | Basis weight (g/m²) | - | - | - | - | - | - | - | - | 120 | 100 |
| | Weight (g) | - | - | - | - | - | - | - | - | 0.288 | 0.24 |
| Total water absorbing polymer | Basis weight (g/m²) | 80 | 100 | 120 | 140 | 80 | 100 | 120 | 140 | 120 | 120 |
| Maximum temperature | °C | 69.8 | 66.3 | 68.0 | 68.5 | 72.4 | 72.0 | 70.1 | - | 75.8 | 75.1 |
| 35→45°C | Minute | 2.6 | 1.4 | 1.2 | 1.2 | 2.1 | 1.4 | 1.4 | - | 0.9 | 1.0 |
| Temperature rise time | | | | | | | | | | | |
| Amount of steam for 10 minutes | mg | 317 | 463 | 519 | 528 | 430 | 477 | 485 | - | 518 | 465 |

| | | | | | | | | | | | |
|---|---|---|---|---|---|---|---|---|---|---|---|
| *1: The value is a total basis weight obtained by summing the basis weight of the second water absorbing polymer and the basis weights of the first and second water absorbing sheets. Here, in Nos. 9 and 10, the basis weight of the water absorbing polymer contained in the polymer sheet was calculated as the basis weight of the second water absorbing polymer. *2: In Nos. 9 and 10, the value is a total basis weight obtained by summing the basis weight of the water absorbing polymer in the polymer sheet laminated on the polymer sheet among two polymer sheets and the basis weights of two water absorbing sheets. *3: The polymer sheet means a polymer sheet laminated to be in contact with the heat generating layer among two polymer sheets. | | | | | | | | | | | |

**[Table 3]**

| Condition | | 11 | 12 | 13 | 14 | 15 | 16 | 17 | 18 | 19 |
|---|---|---|---|---|---|---|---|---|---|---|
| Configuration | First water absorbent layer | Present | Present | Present | Present | Present | Present | Present | Present | Present |
| | Water absorbing sheet | Present | Present | Present | Present | Present | Present | Present | Present | Present |
| | Second water absorbent layer | Present | Present | Present | Present | Present | Present | Present | Present | Present |
| Spray amount of first water absorbing polymer | Basis weight (g/m²) | 30 | 50 | 70 | 90 | 110 | 50 | 70 | 90 | 110 |
| | Weight (g) | 0.072 | 0.120 | 0.168 | 0.216 | 0.264 | 0.120 | 0.168 | 0.216 | 0.264 |
| PS^{*2} | Basis weight (g/m2)^{*1} | 120 | 120 | 120 | 120 | 120 | 100 | 100 | 100 | 60 |
| | Weight (g) | 0.288 | 0.288 | 0.388 | 0.288 | 0.288 | 0.240 | 0.240 | 0.240 | 0.144 |
| PS^{*3} on heat generating layer | Basis weight (g/m²) | - | - | - | - | - | - | - | - | - |
| | Weight (g) | - | - | - | - | - | - | - | - | - |
| Total water absorbing polymer | Basis weight (g/m²) | 100 | 120 | 140 | 160 | 180 | 100 | 120 | 140 | 120 |
| Maximum temperature | °C | 74.6 | 74.3 | 74.8 | 74.2 | 74.5 | 75.2 | 74.3 | 72.7 | 70.3 |
| 35→45°C | Minute | 1.0 | 0.9 | 0.8 | 0.8 | 0.8 | 1.0 | 0.9 | 0.8 | 0.9 |
| Temperature rise time | | | | | | | | | | |
| Amount of steam for 10 minutes | mg | 537 | 597 | 588 | 591 | 584 | 530 | 600 | 590 | 554 |

| | | | | | | | | | | |
|---|---|---|---|---|---|---|---|---|---|---|
| *1: The value is a total basis weight obtained by summing the basis weight of the second water absorbing polymer and the basis weights of the first and second water absorbing sheets. | | | | | | | | | | |

Here, in Nos. 9 and 10, the basis weight of the water absorbing polymer contained in the polymer sheet was calculated as the basis weight of the second water absorbing polymer.
*2: In Nos. 9 and 10, the value is a total basis weight obtained by summing the basis weight of the water absorbing polymer in the polymer sheet laminated on the polymer sheet among two polymer sheets and the basis weights of two water absorbing sheets.
*3: The polymer sheet means a polymer sheet laminated to be in contact with the heat generating layer among two polymer sheets.

As shown in Nos. 11 to 19 in the above table, 530 mg or more of steam can be generated for 10 minutes by providing the first water absorbent layer, the water absorbing sheet, and the second water absorbent layer on the heat generating layer. In addition, the maximum temperature of the steam generating sheet is as high as 70°C or higher, and the temperature rises quickly when the temperature rise time from 35°C to 45°C is 1.0 minute or shorter.

In particular, when the basis weight of the water absorbing polymer in the first water absorbent layer and the basis weight of the water absorbing polymer in the polymer sheet are appropriately selected, the amount of steam generated can be further increased, and the temperature rise time can be further shortened.

When the second water absorbent layer is not provided (Nos. 1 to 4), the maximum temperature is lower than 70°C, and 530 mg or more of the steam cannot be generated in 10 minutes. In addition, when the first water absorbent layer is not provided (Nos. 5 to 7), it takes time to raise the temperature and the amount of steam generated is less than 500 mg. Even in a case where a plurality of water absorbing sheets and second water absorbent layers are provided, when the first water absorbent layer is not provided (Nos. 9 and 10), 530 mg or more of steam cannot be generated in 10 minutes.

In addition, when the heating tool (steam cup) of Fig. 4 was produced using the steam generator of the present invention, sufficient and comfortable steam was supplied to a nasal cavity in a short time.

### Reference Signs List

- 10: Steam generating sheet
- 11: Substrate layer
- 12: Heat generating layer
- 12a: Composition layer
- 14: First water absorbent layer
- 14a: Particles of first water absorbing polymer
- 15: Crepe paper
- 16: Water-retention layer
- 17: First water absorbing sheet
- 18: Second water absorbent layer
- 18a: Particles of second water absorbing polymer
- 19: Second water absorbing sheet
- 20: Steam generator
- 21: First sheet
- 22: Second sheet
- 30: Heating tool
- 31: Main body portion
- 32: First panel
- 33: Second panel
- 32A, 32B, 32C: Side edge
- 33A, 33B, 33C: Side edge
- 34, 35: Storage portion
- D: Boundary line
- 100, 101, 102: Steam generating sheet

## Claims

1. A steam generator (20) comprising:
a heat generating layer (12) comprising an oxidizable metal, a carbon component, and water;
a first water absorbent layer (14) laminated on the heat generating layer (12) and comprising a first water absorbent;
a water absorbing sheet (17) laminated on the first water absorbent layer (14); and
a second water absorbent layer (18) laminated on the water absorbing sheet (17) and comprising a second water absorbent.

2. The steam generator (20) according to claim 1, wherein the first water absorbent comprises a first water absorbing polymer.

3. The steam generator (20) according to claim 2, wherein a basis weight of the first water absorbing polymer in the first water absorbent layer (14) is 15 g/m² or more.

4. The steam generator (20) according to claim 2 or 3, wherein a basis weight of the first water absorbing polymer in the first water absorbent layer (14) is 300 g/m² or less.

5. The steam generator (20) according to any one of claims 1 to 4, further comprising a second water absorbing sheet (19) laminated on the second water absorbent layer (18).

6. The steam generator (20) according to any one of claims 1 to 5, further comprising:
a first sheet (21) provided on the second water absorbent layer side and having a degree of air permeability of 200 sec/100 mL or less; and
a second sheet (22) provided on the heat generating layer side and having a degree of air permeability of more than 8,000 sec/100 mL,
wherein peripheries of the first sheet (21) and the second sheet (22) are sealed to form a bag.

7. A heating tool (30) comprising:
the steam generator (20) according to claim 6; and
a main body portion (31) storing the steam generator and covering a mouth and a nose of a user.

8. A method for producing a steam generator (20) comprising a heat generating layer (12) comprising an oxidizable metal, a carbon component, and water, a first water absorbent layer (14) laminated on the heat generating layer (12) and comprising a first water absorbent, a water absorbing sheet (17) laminated on the first water absorbent layer (14), and a second water absorbent layer (18) laminated on the water absorbing sheet (17) and comprising a second water absorbent,
the method comprising:
laminating the first water absorbent layer (14) comprising the first water absorbent on the heat generating layer (12);
laminating the water absorbing sheet (17) on the first water absorbent layer (14); and
laminating the second water absorbent layer (18) comprising the second water absorbent on the water absorbing sheet (17).

9. The method for producing a steam generator (20) according to claim 8, wherein the first water absorbent layer (14) is obtained by spraying a first water absorbing polymer.

10. The method for producing a steam generator (20) according to claim 9, wherein the first water absorbing polymer is sprayed at a basis weight of 15 g/m² or more.

11. The method for producing a steam generator (20) according to claim 9 or 10, wherein the first water absorbing polymer is sprayed at a basis weight of 300 g/m² or less.

12. The method for producing a steam generator (20) according to any one of claims 8 to 11, further comprising laminating a second water absorbing sheet (19) on the second water absorbent layer (18).

13. The method for producing a steam generator (20) according to any one of claims 8 to 12, further comprising:
providing a first sheet (21) having a degree of air permeability of 200 sec/100 mL or less on the second water absorbent layer side;
providing a second sheet (22) having a degree of air permeability of more than 8,000 sec/100 mL on the heat generating layer side; and
sealing peripheries of the first sheet (21) and the second (22) sheet to form a bag.

## Patentansprüche

1. Dampferzeuger (20), umfassend:
eine wärmeerzeugende Schicht (12), umfassend ein oxidierbares Metall, eine Kohlenstoffkomponente und Wasser;
eine erste wasserabsorbierende Schicht (14), die auf die wärmeerzeugende Schicht (12) laminiert ist und ein erstes Wasserabsorptionsmittel umfasst;
ein wasserabsorbierendes Sheet (17), das auf die erste wasserabsorbierende Schicht (14) laminiert ist; und
eine zweite wasserabsorbierende Schicht (18), die auf das wasserabsorbierende Sheet (17) laminiert ist und ein zweites Wasserabsorptionsmittel umfasst.

2. Dampferzeuger (20) gemäß Anspruch 1, wobei das erste Wasserabsorptionsmittel ein erstes wasserabsorbierendes Polymer umfasst.

3. Dampferzeuger (20) gemäß Anspruch 2, wobei ein Flächengewicht des ersten wasserabsorbierenden Polymers in der ersten wasserabsorbierenden Schicht (14) 15 g/m² oder mehr beträgt.

4. Dampferzeuger (20) gemäß Anspruch 2 oder 3, wobei ein Flächengewicht des ersten wasserabsorbierenden Polymers in der ersten wasserabsorbierenden Schicht (14) 300 g/m² oder weniger beträgt.

5. Dampferzeuger (20) gemäß einem der Ansprüche 1 bis 4, ferner umfassend ein zweites wasserabsorbierendes Sheet (19), das auf die zweite wasserabsorbierende Schicht (18) laminiert ist.

6. Dampferzeuger (20) gemäß einem der Ansprüche 1 bis 5, ferner umfassend:
ein erstes Sheet (21), das auf der Seite der zweiten wasserabsorbierenden Schicht angeordnet ist und einen Luftdurchlässigkeitsgrad von 200 sec/100 mL oder weniger aufweist; und
ein zweites Sheet (22), das auf der Seite der wärmeerzeugenden Schicht angeordnet ist und einen Luftdurchlässigkeitsgrad von mehr als 8.000 sec/100 mL aufweist,
wobei die Ränder des ersten Sheets (21) und des zweiten Sheets (22) unter Bildung einer Tasche abgedichtet sind.

7. Heizgerät (30), umfassend:
den Dampferzeuger (20) gemäß Anspruch 6; und
einen Hauptkörperabschnitt (31), der den Dampferzeuger aufnimmt und einen Mund und eine Nase eines Benutzers bedeckt.

8. Verfahren zur Herstellung eines Dampfgenerators (20), umfassend eine wärmeerzeugende Schicht (12), die ein oxidierbares Metall, eine Kohlenstoffkomponente und Wasser umfasst, eine erste wasserabsorbierende Schicht (14), die auf die wärmeerzeugende Schicht (12) laminiert ist und ein erstes Wasserabsorptionsmittel umfasst, ein wasserabsorbierendes Sheet (17), das auf die erste wasserabsorbierende Schicht (14) laminiert ist, und eine zweite wasserabsorbierende Schicht (18), die auf das wasserabsorbierende Sheet (17) laminiert ist und ein zweites Wasserabsorptionsmittel umfasst,
wobei das Verfahren umfasst:
das Laminieren der ersten wasserabsorbierenden Schicht (14), umfassend das erste Wasserabsorptionsmittel, auf die wärmeerzeugende Schicht (12);
das Laminieren des wasserabsorbierenden Sheets (17) auf die erste wasserabsorbierende Schicht (14); und
das Laminieren der zweiten wasserabsorbierenden Schicht (18), umfassend das zweite Wasserabsorptionsmittel, auf das wasserabsorbierende Sheet (17).

9. Verfahren zur Herstellung eines Dampferzeugers (20) gemäß Anspruch 8, wobei die erste wasserabsorbierende Schicht (14) durch Aufsprühen eines ersten wasserabsorbierenden Polymers erhalten wird.

10. Verfahren zur Herstellung eines Dampferzeugers (20) gemäß Anspruch 9, wobei das erste wasserabsorbierende Polymer mit einem Flächengewicht von 15 g/m² oder mehr aufgesprüht wird.

11. Verfahren zur Herstellung eines Dampferzeugers (20) gemäß Anspruch 9 oder 10, wobei das erste wasserabsorbierende Polymer mit einem Flächengewicht von 300 g/m² oder weniger aufgesprüht wird.

12. Verfahren zur Herstellung eines Dampferzeugers (20) gemäß einem der Ansprüche 8 bis 11, ferner umfassend das Laminieren eines zweiten wasserabsorbierende Sheets (19) auf die zweite wasserabsorbierende Schicht (18).

13. Verfahren zur Herstellung eines Dampferzeugers (20) gemäß einem der Ansprüche 8 bis 12, ferner umfassend:
das Anordnen eines ersten Sheets (21) mit einem Luftdurchlässigkeitsgrad von 200 sec/100 mL oder weniger auf der Seite der zweiten wasserabsorbierenden Schicht;
das Anordnen eines zweiten Sheets (22) mit einem Luftdurchlässigkeitsgrad von mehr als 8.000 sec/100 mL auf der Seite der wärmeerzeugenden Schicht; und
das Abdichten der Ränder des ersten Sheets (21) und des zweiten Sheets (22), um eine Tasche zu bilden.

## Revendications

1. Générateur de vapeur (20) comprenant :
une couche de génération de chaleur (12) comprenant un métal oxydable, un composant de carbone, et de l'eau ;
une première couche absorbante d'eau (14) stratifiée sur la couche de génération de chaleur (12) et comprenant un premier absorbant d'eau ;
une feuille d'absorption d'eau (17) stratifiée sur la première couche absorbante d'eau (14) ; et
une seconde couche absorbante d'eau (18) stratifiée sur la feuille d'absorption d'eau (17) et comprenant un second absorbant d'eau.

2. Générateur de vapeur (20) selon la revendication 1, dans lequel le premier absorbant d'eau comprend un premier polymère absorbant d'eau.

3. Générateur de vapeur (20) selon la revendication 2, dans lequel un poids de base du premier polymère absorbant d'eau dans la première couche absorbante d'eau (14) est de 15 g/m² ou plus.

4. Générateur de vapeur (20) selon la revendication 2 ou 3, dans lequel un poids de base du premier polymère absorbant d'eau dans la première couche absorbante d'eau (14) est de 300 g/m² ou moins.

5. Générateur de vapeur (20) selon l'une quelconque des revendications 1 à 4, comprenant en outre une seconde feuille d'absorption d'eau (19) stratifiée sur la seconde couche absorbante d'eau (18).

6. Générateur de vapeur (20) selon l'une quelconque des revendications 1 à 5, comprenant en outre :
une première feuille (21) prévue sur le côté de la seconde couche absorbante d'eau et présentant un degré de perméabilité d'air de 200 sec/100 mL ou moins ; et
une seconde feuille (22) prévue le côté de la couche de génération de chaleur et présentant un degré de perméabilité d'air de plus de 8000 sec/100 mL,
dans lequel les périphéries de la première feuille (21) et de la seconde feuille (22) sont scellées pour former un sac.

7. Outil de chauffage (30) comprenant :
le générateur de vapeur (20) selon la revendication 6 ; et
une partie de corps principal (31) stockant le générateur de vapeur et recouvrant une bouche et un nez d'un utilisateur.

8. Procédé de production d'un générateur de vapeur (20) comprenant une couche de génération de chaleur (12) comprenant un métal oxydable, un composant de carbone, et de l'eau, une première couche absorbante d'eau (14) stratifiée sur la couche de génération de chaleur (12) et comprenant un premier absorbant d'eau, une feuille d'absorption d'eau (17) stratifiée sur la première couche absorbante d'eau (14), et une seconde couche absorbante d'eau (18) stratifiée sur la feuille d'absorption d'eau (17) et comprenant un second absorbant d'eau
le procédé comprenant :
une stratification de la première couche absorbante d'eau (14) comprenant le premier absorbant d'eau sur la couche de génération de chaleur (12) ;
une stratification de la feuille d'absorption d'eau (17) sur la première couche absorbante d'eau (14) ; et
une stratification de la seconde couche absorbante d'eau (18) comprenant le second absorbant d'eau sur la feuille d'absorption d'eau (17).

9. Procédé de production d'un générateur de vapeur (20) selon la revendication 8, dans lequel la première couche absorbante d'eau (14) est obtenue par pulvérisation d'un premier polymère absorbant d'eau.

10. Procédé de production d'un générateur de vapeur (20) selon la revendication 9, dans lequel le premier polymère absorbant d'eau est pulvérisé à un poids de base de 15 g/m² ou plus.

11. Procédé de production d'un générateur de vapeur (20) selon la revendication 9 ou 10, dans lequel le premier polymère absorbant d'eau est pulvérisé à un poids de base de 300 g/m² ou moins.

12. Procédé de production d'un générateur de vapeur (20) selon l'une quelconque des revendications 8 à 11, comprenant en outre une stratification d'une seconde feuille d'absorption d'eau (19) sur la seconde couche absorbante d'eau (18).

13. Procédé de production d'un générateur de vapeur (20) selon l'une quelconque des revendications 8 à 12, comprenant en outre :
une fourniture d'une première feuille (21) présentant un degré de perméabilité d'air de 200 sec/100 mL ou moins sur le côté de la seconde couche absorbante d'eau ;
une fourniture d'une seconde feuille (22) présentant un degré de perméabilité d'air de plus de 8000 sec/100 mL sur le côté de la couche de génération de chaleur ; et
le fait de sceller les périphéries de la première feuille (21) et de la seconde feuille (22) pour former un sac.
